(19) Europäisches Patentamt
European Patent Office
Office européen des brevets

(11) Veröffentlichungsnummer: **0 048 378**
**B1**

(12) # EUROPÄISCHE PATENTSCHRIFT

(45) Veröffentlichungstag der Patentschrift: **30.05.84**

(51) Int. Cl.³: **C 07 C 1/04,** C 07 C 11/02 // B01J23/84

(21) Anmeldenummer: **81107022.6**

(22) Anmeldetag: **07.09.81**

(54) **Verfahren zur Herstellung ungesättigter Kohlenwasserstoffe.**

(30) Priorität: **19.09.80 DE 3035404**

(43) Veröffentlichungstag der Anmeldung: **31.03.82 Patentblatt 82/13**

(45) Bekanntmachung des Hinweises auf die Patenterteilung: **30.05.84 Patentblatt 84/22**

(84) Benannte Vertragsstaaten: **FR GB NL**

(56) Entgegenhaltungen:
**DE - A - 2 438 251**
**DE - A - 2 507 647**
**DE - B - 2 518 982**
**DE - C - 764 165**
**DE - C - 865 893**
**DE - C - 904 891**
**US - A - 2 486 633**

(73) Patentinhaber: **Ruhrchemie Aktiengesellschaft, Bruchstrasse 219, D-4200 Oberhausen 13 (DE)**

(72) Erfinder: **Büssemeier, Bernd, Dr., Dipl.-Chem., Nikolaus-Ehlen-Strasse 28, D-4330 Mülheim/Ruhr (DE)**
Erfinder: **Cornils, Boy, Dr. Dipl.-Chem., Friedrich-Ebert-Strasse 45, D-4220 Dinslaken (DE)**
Erfinder: **Frohning, Carl Dieter, Dr., Dipl.-Chem., Eisenbruch 1, D-4200 Oberhausen 13 (DE)**

(74) Vertreter: **Reichelt, Karl-Heinz, Dr. et al, m. Br. Ruhrchemie Aktiengesellschaft Abt. PLD Postfach 13 01 60, D-4200 Oberhausen 13 (DE)**

EP 0 048 378 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft die Herstellung von kurzkettigen Olefinen aus Synthesegas.

Bei der Hydrierung von Kohlenoxiden zu Olefinen ist der Umsatz in hohem Masse vom Wasserstoffpartialdruck abhängig. Je grösser der Wasserstoffpartialdruck ist, desto höhere Umsätze werden erzielt. Gleichzeitig nimmt jedoch mit steigendem Wasserstoffpartialdruck auch die Hydrierung der primär gebildeten Olefine zu. Daneben ist noch die Konvertierung des Kohlenmonoxids durch das bei der Synthese gebildete Reaktionswasser entsprechend

$$H_2O + CO \rightleftarrows H_2 + CO_2$$

zu berücksichtigen, die unter Einwirkung der für die Kohlenoxidhydrierung verwendeten Katalysatoren abläuft. Dadurch geht ein nicht unerheblicher Anteil des eingesetzten Kohlenmonoxids verloren. In der Praxis stellt sich daher die Aufgabe, in Gegenwart selektiv wirkender Katalysatoren den Synthesedruck bzw. den Wasserstoffpartialdruck so einzustellen, dass die Kohlenoxidhydrierung mit hohem Umsatz abläuft und sowohl die Hydrierung der primär gebildeten Olefine als auch die Konvertierungsreaktion zwischen Kohlenmonoxid und Wasser weitgehend unterbunden werden.

Die Herstellung ungesättigter, insbesondere gasförmiger Kohlenwasserstoffe durch Umsetzung von Kohlenoxid mit Wasserstoff in Gegenwart von Katalysatoren ist schon mehrfach beschrieben worden. So wird nach dem Verfahren der DE-PS 922 883 mit Eisen-Schmelzkatalysatoren gearbeitet, die periodisch oder kontinuierlich aus dem Reaktionsraum entfernt, regeneriert, reduziert und wieder zurückgeführt werden. Die Umsetzung wird bei gewöhnlichem oder leicht erhöhtem Druck und bei Temperaturen oberhalb etwa 450 °C, zweckmässigerweise bei 470 bis 600 °C durchgeführt.

Nach einer anderen, in der DE-PS 896 338 beschriebenen Arbeitsweise gewinnt man ungesättigte, gasförmige Kohlenwasserstoffe durch Umsetzung von Kohlenmonoxid mit Wasserstoff in Gegenwart beständiger Oxide der Metalle der II. bis VII. Gruppe des Periodensystems. Die Reaktion erfolgt bei etwa Atmosphärendruck und bei Temperaturen oberhalb 520 °C.

Zur Herstellung von Ethylen durch Hydrierung von Kohlenmonoxid mit Wasserstoff können nach DE-AS 1 271 098 auch Katalysatoren eingesetzt werden, die zu mindestens 98 Gew.-% aus einem Träger und zu 0,3 bis 2 Gew.-% aus Kobalt, Nickel oder Platin bestehen. Die Umsetzung erfolgt mit einer Drucksatzgeschwindigkeit von 2500 bis 3000 Liter Gas je Liter Katalysator und Stunde bei Temperaturen von 300 bis 450 °C und Drücken von 130 bis 200 mm Quecksilbersäure. Das Verfahren zeichnet sich durch gute Selektivität zu niederen gasförmigen Olefinen aus, jedoch befriedigen die erreichbaren Synthesegas-Umsätze – sie liegen in der Grössenordnung von 10 bis 20% – nicht.

Eine deutliche Verbesserung des Umsatzes der Ausgangsstoffe und der Olefinausbeute wird mit Katalysatoren erzielt, die aus Eisen und/oder Kobalt und daneben Titan oder Thorium zusammengesetzt sind (vgl. DE-PS 25 36 488).

Nach der DE-PS 25 18 964 gelingt auch mit Eisenkatalysatoren, die Vanadium oder Mangan und ausserdem ZnO und $K_2O$ enthalten, die Herstellung ungesättigter Kohlenwasserstoffe, insbesondere gasförmiger Olefine durch Hydrierung von Kohlenoxiden mit Wasserstoff in hohen Ausbeuten und mit hoher Selektivität.

Aus der DE-OS 24 38 251 ist ein zweistufiges Verfahren zur Gewinnung von Benzin, also gesättigten Kohlenwasserstoffen aus Synthesegas bekannt. Hierbei wird zunächst an einem Gemisch aus Methanolsynthese-Katalysator und Dehydratisierungskatalysator ein Dimethyläther enthaltendes Produkt hergestellt. In der zweiten Stufe erfolgt dann an kristallinem Aluminosilikatszeolithen als Katalysator die Umwandlung des Äthers in die flüssigen Kohlenwasserstoffe.

In der US-PS 24 86 633 ist ein Prozess zur Herstellung von Kohlenwasserstoffen mit mehr als einem Kohlenstoffatom im Molekühl aus Synthesegas beschrieben. Die Umsetzung erfolgt in Gegenwart von Katalysatoren. Zur Regelung der Temperatur führt man in die Reaktionszone einen flüssigen Alkohol ein, dessen Hauptaufgabe es ist, die überschüssige Wärme abzuführen, die bei der exothermen Kohlenwasserstoffsynthese auftritt.

Die bei den bekannten Verfahren angewandten hohen Reaktionstemperaturen haben entsprechend dem Boudouard-Gleichgewicht die Bildung von Kohlenstoff aus Kohlenmonoxid zur Folge. Die Kohlenstoffabscheidung führt zur Desaktivierung durch Belegung der Katalysatoroberfläche und im Einzelfall zu einer Sprengung des Katalysatorgefüges, wodurch die Lebensdauer des Katalysators beträchtlich herabgesetzt wird.

Ein weiterer Nachteil der bisher bekannten Verfahren zur Gewinnung von Olefinen aus Synthesegas ist die nicht immer befriedigende Standzeit der Katalysatoren. Es hat sich nämlich gezeigt, dass häufig mit zunehmender Betriebsdauer die Selektivität bezüglich der Olefinbildung abnimmt und die Lebensdauer der Katalysatoren, begünstigt durch hohe CO-Partialdrücke und hohe Reaktionstemperaturen, häufig auf wenig hundert Stunden begrenzt.

Es bestand daher die Aufgabe, ein Verfahren zu entwickeln, das die aufgezeigten Nachteile überwindet und die selektive Bildung von Olefinen aus Synthesegas mit hohen Umsätzen und Ausbeuten und langer Lebensdauer der Katalysatoren gewährleistet.

Erfindungsgemäss werden ungesättigte Kohlenwasserstoffe, insbesondere Olefine mit 2 bis 4 Kohlenstoffatomen durch katalytische Hydrierung von Kohlenoxiden mit Wasserstoff bei 220 bis 500 °C und Drücken bis 30 bar erhalten, wenn

die Umsetzung in Gegenwart Eisen und/oder Kobalt enthaltender Katalysatoren unter Zusatz von dampfförmigen niedermolekularen aliphatischen Alkoholen zum Kohlenoxid-Wasserstoff-Gemisch erfolgt.

Überraschenderweise hat sich gezeigt, dass nach dem neuen Verfahren durch Zusatz von dampfförmigen niedermolekularen Alkoholen zum Synthesegas die Bildung kurzkettiger Olefine synergistisch verstärkt wird. Die Olefinausbeute ist bei Einsatz von Synthesegas und Alkoholen nämlich deutlich höher als die Summe der Olefinmengen, die unter den selben Reaktionsbedingungen mit Synthesegas allein oder mit Alkoholen allein gebildet werden. Darüber hinaus wird die Lebensdauer der erfindungsgemäss eingesetzten Katalysatoren bedeutend erhöht.

Die im Rahmen der neuen Arbeitsweise verwendeten und an sich bekannten Katalysatoren können aus Eisen und/oder Kobalt als wesentliche Komponente bestehen. Auch andere Metalle der VIII. Gruppe des Periodensystems der Elemente, wie Nickel, Iridium, Palladium oder Platin, können verwendet werden, doch ist ihre Bedeutung geringer, da sie Olefine nur in untergeordneter Menge liefern.

Daneben können die Katalysatoren Aktivatoren enthalten, die die Ausbildung einer grossen Oberfläche bewirken und Rekristallisationsvorgänge in der katalytisch aktiven Phase verhindern. Überdies können Aktivatoren die Selektivität beeinflussen. Als Aktivatoren finden vornehmlich Oxide von Metallen der Gruppen IIa und/oder IIb des Periodensystems der Elemente Anwendung, insbesondere MgO, CaO und ZnO, die in der Menge von 2 bis 20 Gew.-% (bezogen auf die Katalysatormasse) eingesetzt werden. Auch Alkalimetallcarbonate wie $NaCO_3$ oder $K_2CO_3$ sind gebräuchliche Aktivatoren. Zur Verbesserung der Selektivität der Katalysatoren hinsichtlich der Bildung von Olefinen mit 2 bis 4 Kohlenstoffatomen hat sich der Zusatz von schwerreduzierbaren Oxiden des Vanadiums und/oder Mangans in Mengen von 5 bis 50 Gew.-% (bezogen auf die Katalysatormasse) sowie von Titan oder Thorium in Mengen von 5 bis 50 Gew.-% (bezogen auf die Katalysatormasse) sehr bewährt.

Die Herstellung der Katalysatoren erfolgt durch Fällung der Bestandteile aus ihren wässrigen Lösungen mit geeigneten Fällungsreagenzien wie Alkalimetallcarbonaten. Ein anderes Herstellungsverfahren besteht in der Mischung und Homogenisierung der Bestandteile und anschliessende Formung der Masse. Ferner können die Katalysatoren auch durch Sintern der gemischten Komponente erhalten werden.

Als niedermolekulare aliphatische Alkohole, die nach der erfindungsgemässen Arbeitsweise mit dem Gemisch aus Kohlenoxiden und Wasserstoff über den Katalysator geleitet werden, kommen insbesondere Alkohole mit 1 bis 3 Kohlenstoffatomen in Betracht, d.h. Methanol, Äthanol sowie die isomeren Propanole. Sie können allein oder in Gemischen verwendet werden. Das Mischungsverhältnis von Kohlenoxiden und Wasserstoff zu Alkohol kann in weiten Bereichen variiert werden. Bewährt hat es sich, auf 1 bis 4 Volumenteile Kohlenoxid-Wasserstoff-Gemisch 1 Volumenteil dampfförmigen Alkohol einzusetzen.

Unter dem Begriff Kohlenoxide werden Kohlenmonoxid und Kohlendioxid oder deren Gemische verstanden. Üblicherweise geht man zur Herstellung der Olefine nach dem erfindungsgemässen Verfahren von Synthesegas, d.h. dem Gemisch von Kohlenmonoxid und Wasserstoff, aus, das aus minderwertigen Erdölfraktionen, insbesondere aber auch aus Kohle durch partielle Oxidation in Gegenwart von Wasserdampf nach bekannten Verfahren zugänglich ist.

Im Normalfall enthält das Synthesegas gleiche Volumenteile Kohlenmonoxid und Wasserstoff. Es können auch kohlenoxid- oder wasserstoffreiche Gemische mit Kohlenoxid- zu -Wasserstoff-Anteilen von 30:70 bis 70:30 (in Molen) eingesetzt werden.

Die Durchführung des neuen Verfahrens gestaltet sich einfach und ist an keine besondere Arbeitsweise gebunden. Die Katalysatoren werden im allgemeinen in Form eines Festbettes angeordnet. Sie können jedoch auch nach anderen Verfahrensvarianten, z.B. in feinverteilter Form als Wirbelschicht, eingesetzt werden.

Zur Umsetzung werden die Ausgangsstoffe Synthesegas und Alkohol bei Temperaturen von 220 bis 500 °C, insbesondere 250 bis 350 °C und Drücker bis 30 bar, vorzugsweise 10 bis 30 bar über den Katalysator geleitet. Das den Reaktionsraum verlassende Gasgemisch wird zweckmässigerweise nach Entfernung der gebildeten ungesättigten gasförmigen Kohlenwasserstoffe und nach Ergänzung des verbrauchten Alkohols der Apparatur ganz oder teilweise wieder zugeführt.

Die Erfindung wird durch die nachfolgenden Beispiele näher erläutert, sie ist jedoch nicht auf die Ausführungsform dieser Beispiele beschränkt.

Alle Versuche werden in einem Rohrreaktor von 150 cm Länge und 2 cm lichter Weite durchgeführt. In den Reaktor wird ein Gemisch aus 50 ml Katalysator und 250 ml Siliciumcarbid SiC, das gute Wärmeleitfähigkeit aufweist, eingefüllt. Die Höhe der Katalysatorschüttung beträgt 96 cm. Nachdem der Reaktor mittels einer elektrischen Heizung auf die Reaktionstemperatur gebracht ist, werden die Einsatzstoffe über den Katalysator geleitet.

Die in den Versuchen eingesetzten Katalysatoren haben folgende Zusammensetzung:

Beispiele 1 bis 3
100 Gew.-teile Eisen in Form von $Fe_2O_3$
80 Gew.-teile Vanadium in Form von $V_2O_5$
30 Gew.-teile ZnO und 4 Gew.-teile $K_2O$

Beispiel 4
100 Gew.-teile Eisen in Form von $Fe_2O_3$
80 Gew.-teile Vanadium in Form von $V_2O_5$
7 Gew.-teile MgO und 4 Gew.-teile $K_2O$

Die Herstellung der Katalysatoren erfolgt durch einfaches Mischen und homogenisieren der pulverförmigen Bestandteile.

Beispiel 1

| Einsatzprodukte | | Synthesegas | Synthesegas + Methanol | Stickstoff + Methanol |
|---|---|---|---|---|
| Katalysator | | ZnO-haltiger | Fe-Katalysator | |
| Temperatur | (°C) | 335 | 335 | 330 |
| Druck | (bar) | 10 | 10 | 10 |
| $CO + H_2$-Einsatz | (V/Vh) | 1005 | 944 | – |
| $N_2$-Einsatz | (V/Vh) | – | – | 980 |
| Alkohol (Gas)-Einsatz | (V/Vh) | – | 562 | 591 |
| Ausbeute | ($g/Nm^3$) | | | |
| Methan | ($g/Nm^3$) | 41,8 | 64,1 | 19,6 |
| Ethan | ($g/Nm^3$) | 24,7 | 25,5 | Spuren |
| Ethylen | ($g/Nm^3$) | 2,8 | 13,0 | Spuren |
| Propan | ($g/Nm^3$) | 30,3 | 37,2 | Spuren |
| Propylen | ($g/Nm^3$) | 22,7 | 39,0 | Spuren |
| Butane | ($g/Nm^3$) | 8,8 | 8,7 | Spuren |
| Butene | ($g/Nm^3$) | 15,2 | 24,8 | Spuren |
| $C_2$–$C_4$-Olefine | ($g/Nm^3$) | 40,7 | 76,8 | |

Beispiel 2

| Einsatzprodukte | | Synthese-Gas | Synthese-Gas + Ethanol | Stickstoff + Ethanol |
|---|---|---|---|---|
| Katalysator | | ZnO-haltiger | Fe-Katalysator | |
| Temperatur | (°C) | 330 | 330 | 330 |
| Druck | (bar) | 10 | 10 | 10 |
| $CO + H_2$-Einsatz | (V/Vh) | 839 | 920 | – |
| $N_2$-Einsatz | (V/Vh) | – | – | 933 |
| Alkohol (Gas)-Einsatz | (V/Vh) | – | 365 | 397 |
| Ausbeute | ($g/Nm^3$) | | | |
| Methan | ($g/Nm^3$) | 14,6 | 11,5 | 0,7 |
| Ethan | ($g/Nm^3$) | 2,9 | 18,7 | 3,0 |
| Ethylen | ($g/Nm^3$) | 12,6 | 21,3 | 3,7 |
| Propan | ($g/Nm^3$) | 2,6 | 3,0 | 0,2 |
| Propylen | ($g/Nm^3$) | 18,8 | 28,7 | 0,9 |
| Butane | ($g/Nm^3$) | 1,7 | 1,6 | 0,2 |
| Butene | ($g/Nm^3$) | 13,1 | 24,7 | 5,6 |
| $C_2$–$C_4$-Olefine | ($g/Nm^3$) | 44,5 | 74,7 | 10,2 |

Beispiel 3

| Einsatzprodukte | | Synthese-Gas | Synthese-Gas + Isopropanol | Stickstoff + Isopropanol |
|---|---|---|---|---|
| Katalysator | | ZnO-haltiger | Fe-Katalysator | |
| Temperatur | (°C) | 330 | 330 | 330 |
| Druck | (bar) | 10 | 10 | 10 |
| $CO + H_2$-Einsatz | (V/Vh) | 839 | 866 | – |
| $N_2$-Einsatz | (V/Vh) | – | – | 850 |
| Alkohol (Gas)-Einsatz | (V/Vh) | – | 288 | 289 |
| Ausbeute | ($g/Nm^3$) | | | |
| Methan | ($g/Nm^3$) | 14,6 | 17,0 | – |
| Ethan | ($g/Nm^3$) | 2,9 | 6,8 | – |
| Ethylen | ($g/Nm^3$) | 12,6 | 9,3 | – |

(Fortsetzung) Beispiel 3

| Einsatzprodukte | | Synthese-Gas | Synthese-Gas + Isopropanol | Stickstoff + Isopropanol |
|---|---|---|---|---|
| Propan | (g/Nm³) | 2,6 | 23,4 | 6,2 |
| Propylen | (g/Nm³) | 18,8 | 35,3 | 7,0 |
| Butane | (g/Nm³) | 1,7 | 1,6 | 0,5 |
| Butene | (g/Nm³) | 13,1 | 11,8 | 3,1 |
| $C_2$–$C_4$-Olefine | (g/Nm³) | 44,5 | 56,4 | 10,1 |

Beispiel 4

| Einsatzprodukte | | Synthese-Gas | Synthese-Gas + Methanol | | | Stickstoff + Methanol |
|---|---|---|---|---|---|---|
| Katalysator | | MgO-haltiger | Fe-Katalysator | | | |
| Temperatur | (°C) | 340 | 340 | 340 | 340 | 340 |
| Druck | (bar) | 10 | 10 | 10 | 12 | 12,9–14,9 |
| $CO + H_2$-Einsatz | (V/Vh) | 1073 | 1040 | 1068 | 983 | – |
| $N_2$-Einsatz | (V/Vh) | – | – | – | – | ca. 1000 |
| Alkohol (Gas)-Einsatz | (V/Vh) | – | 227 | 585 | 1229 | 603 |
| Ausbeute | (g/Nm³) | | | | | |
| Methan | (g/Nm³) | 29,6 | 31,8 | 41,1 | 38,9 | 13,2 |
| Ethan | (g/Nm³) | 8,1 | 8,0 | 8,0 | 7,6 | 1,6 |
| Ethylen | (g/Nm³) | 14,4 | 18,0 | 23,3 | 24,9 | 6,5 |
| Propan | (g/Nm³) | 5,8 | ~11,6 | 7,4 | 14,3 | 1,2 |
| Propylen | (g/Nm³) | 25,0 | 31,1 | 37,1 | 34,5 | 7,8 |
| Butane | (g/Nm³) | 2,8 | 3,4 | 5,4 | 4,8 | 0,8 |
| Butene | (g/Nm³) | 14,1 | 18,9 | 25,1 | 25,8 | 5,7 |
| $C_2$–$C_4$-Olefine | (g/Nm³) | 53,5 | 68,0 | 85,5 | 85,2 | 20,0 |

## Patentansprüche

1. Verfahren zur Herstellung ungesättigter Kohlenwasserstoffe, insbesondere Olefine mit 2 bis 4 Kohlenstoffatomen durch katalytische Hydrierung von Kohlenoxiden mit Wasserstoff bei 220 bis 500 °C und Drücken bis 30 bar, dadurch gekennzeichnet, dass die Umsetzung in Gegenwart Eisen und/oder Kobalt enthaltender Katalysatoren unter Zusatz von dampfförmigen niedermolekularen aliphatischen Alkoholen zum Kohlenoxid-Wasserstoff-Gemisch erfolgt.

2. Verfahren nach Anspruch 1, dadurch gekennzeichnet, dass als niedermolekulare Alkohole Alkohole mit 1 bis 3 Kohlenstoffatomen zugesetzt werden.

3. Verfahren nach Anspruch 1 und 2, dadurch gekennzeichnet, dass das Verhältnis von Kohlenoxid-Wasserstoff-Gemisch zu dampfförmigem Alkohol 1 bis 4 Volumenteile zu 1 Volumenteil beträgt.

4. Verfahren nach Anspruch 1 bis 3, dadurch gekennzeichnet, dass die Katalysatoren Oxide von Metallen der Gruppen IIa und/oder IIb des Periodensystems der Elemente enthalten.

5. Verfahren nach Anspruch 1 bis 4, dadurch gekennzeichnet, dass die Katalysatoren schwerreduzierbare Oxide des Vanadiums und/oder Mangans oder des Titans oder des Thoriums enthalten.

## Revendications

1. Procédé de préparation d'hydrocarbures insaturés, en particulier d'oléfines en C2–C4, par hydrogénation catalytique d'oxydes de carbones à l'aide d'hydrogène à une température de 220 à 500 °C et des pressions allant jusqu'à 30 bars, caractérisé en ce que la réaction est effectuée en présence de catalyseurs contenant du fer et/ou du cobalt avec adjonction au mélange oxyde de carbone-hydrogène d'alcools aliphatiques à bas poids moléculaire à l'état de vapeurs.

2. Procédé selon la revendication 1, caractérisé en ce que l'on ajoute, en tant qu'alcools à bas poids moléculaire, des alcools en C1–C3.

3. Procédé selon les revendications 1 et 2, caractérisé en ce que les proportions relatives entre le mélange oxyde de carbone-hydrogène et l'alcool à l'état de vapeurs sont de 1 à 4 parties en volume pour une partie en volume.

4. Procédé selon les revendications 1 à 3, caractérisé en ce que les catalyseurs contiennent des

oxydes de métaux des groupes IIa et/ou IIb de la Classification Périodique des éléments.

5. Procédé selon les revendications 1 à 4, caractérisé en ce que les catalyseurs contiennent des oxydes difficiles à réduire du vanadium et/ou du manganèse ou du titane ou du thorium.

## Claims

1. Process for the preparation of unsaturated hydrocarbons, in particular olefins with 2 to 4 carbon atoms, by catalytic hydrogenation of carbon oxides with hydrogen at 220 to 500 °C and pressures of up to 30 bars, characterised in that the conversion is carried out in the presence of catalysts containing iron and/or cobalt vaporous low molecular weight aliphatic alcohols being added to the carbon oxide/hydrogen mixture.

2. Process according to claim 1 characterised in that alcohols with 1 to 3 carbon atoms are used as low molecular weight alcohols.

3. Process according to claims 1 and 2 characterised in that the ratio of the carbon oxide/hydrogen mixture to vaporous alcohol is 1 to 4 parts by volume to 1 part by volume.

4. Process according to claims 1 to 3 characterised in that the catalysts contain oxides of metals of Groups IIa and/or IIb of the periodic system of the elements.

5. Process according to claims 1 to 4 characterised in that the catalysts contain difficultly reducible oxides of vanadium and/or manganese or of titanium or thorium